# EUROPEAN PATENT SPECIFICATION

(11) **EP 2 223 121 B1**
(45) Date of publication and mention of the grant of the patent: **25.07.2012**
(21) Application number: 07861152.2
(22) Date of filing: 20.12.2007
(51) Int. Cl.: G01N 33/68, C12Q 1/68, G01N 33/53

(54) **METHOD AND KIT FOR USE IN THE DIFFERENTIATION OF IBD AND IBS AND FURTHER DISTINCTION BETWEEN DISEASE TYPES OF IBD**
VERFAHREN UND KIT ZUR VERWENDUNG BEI DER DIFFERENZIERUNG VON IBD UND IBS UND DER WEITEREN UNTERSCHEIDUNG ZWISCHEN IBD-KRANKHEITSTYPEN
PROCÉDÉ ET KIT DESTINÉS À UNE UTILISATION DANS LA DIFFÉRENTIATION DE IBD ET IBS ET DANS LA DISTINCTION COMPLÉMENTAIRE ENTRE DIFFÉRENTS TYPES DE MALADIES IBD

(43) Date of publication of application: 01.09.2010
(73) Proprietor: Index Diagnostics AB (publ), 171 77 Stockholm (SE)
(72) Inventor: VON STEIN, Petra, S-194 34 Upplands Väsby (SE); KOUZNETSOV, Nikolai, S-176 72 Järfälla (SE); VON STEIN, Oliver, S-194 34 Upplands Väsby (SE); GIELEN, Alexander, 124 76 Bandhagen (SE)
(74) Representative: Berg, Anna Margareta
(86) International application number: PCT/SE2007/051057
(87) International publication number: WO 2009/082298

(56) References cited:
- WO-A2-2006/015742
- WO-A2-2008/022177
- US-A1- 2004 241 823
- US-A1- 2004 241 823
- VON STEIN P ET AL: "Multigene Analysis Can Discriminate Between Ulcerative Colitis, Crohn's Disease, and Irritable Bowel Syndrome", GASTROENTEROLOGY, ELSEVIER, PHILADELPHIA, PA, vol. 134, no. 7, 1 June 2008 (2008-06-01), pages 1869-1881, XP022756831, ISSN: 0016-5085, DOI: DOI:10.1053/J.GASTRO.2008.02.083 [retrieved on 2008-03-02]
- VON STEIN, P. ET AL: 'Multi-gene approach to discriminate for ulcerative colitis, Crohn's disease and irritable bowel syndrome' GASTROENTEROLOGY vol. 132, no. 4, April 2007, ISSN 0016-5085 page A179
- LAWRANCE, I. ET AL.: 'Ulcerative colitis and Crohn's disease: distinctive gene expression profiles and novel susceptibility candidate genes' HUMAN MOLECULAR GENETICS vol. 10, no. 5, 01 March 2001, pages 445 - 456, XP002955653
- CAMILLERI, M. ET AL. ALTERATIONS IN EXPRESSION OF PLL AND SERT IN MUCOSAL BIOPSY SPECIMENS OF PATIENTS WITH IRRITABLE BOWEL SYNDROME vol. 132, no. 1, January 2007, pages 17 - 25, XP005920657
- NEWELL, K., J. ET AL.: 'Matrilysin (matrix metalloproteinase-7) expression in ulcerative colitis-related tumorigenesis' MOLECULAR CARCINOGENESIS vol. 34, no. 2, June 2002, ISSN 0899-1987 pages 59 - 63
- HARTUPEE, J. C. ET AL.: 'Isolation and characterization of a cDNA encoding a novel member of the human regenerating protein family: Reg IV.' BIOCHIMICA ET BIOPHYSICA ACTA vol. 1518, no. 3, 16 April 2001, ISSN 0006-3002 pages 287 - 293, XP004235048
- ISAACS, K. L. ET AL.: 'Cytokine messenger RNA profiles in inflammatory bowel disease mucosa detected by polymerase chain reaction amplification' GASTROENTEROLOGY vol. 103, no. 5, November 1992, pages 1587 - 1595, XP002969859

## Description

The present invention relates to a method for use in the diagnosis of inflammatory bowel disease using a multi-gene approach. More particularly, the present invention relates to a method for use in the diagnosing and/or differentiating inflammatory bowel disease (IBD) from inflammatory bowel syndrome (IBS) by determining the expression of at least one specific marker gene or genes, e.g. using oligonucleotides specifically amplifying said marker genes, or determining the expression of the corresponding proteins, e.g. using antibodies specifically recognizing the proteins encoded by said specific marker gene or genes. The diagnosing method can be advantageously used in the early diagnosis and differentiation of these diseases due to the improved accuracy and reproducibility.

### Background of the invention

Inflammatory bowel disease (IBD) describes a state of chronic relapsing intestinal inflammation. The current understanding of disease pathogenesis suggests a complex interplay of multiple environmental and genetic factors. It causes significant morbidity in populations of European origin, with the two major forms - Crohn's disease (CD) and ulcerative colitis (UC) - having a combined prevalence of 150-250 / 10000. Both forms of IBD have a peak incidence in early adult life but can develop at any stage. They affect the sexes to an approximately equal extent. Environmental risk factors for the development of IBD are poorly defined. The best characterized factor is smoking, which clearly increases the risk of developing Crohn's disease but protects against ulcerative colitis, for reasons that are not clear.

Disease management is a long-term commitment for both patient and physician, since there is currently no cure for either condition. Approximately 30% of IBD patients undergo surgery during their lifetime and patients with long-standing IBD are at considerable risk of developing colorectal cancer. Three out of ten IBD patients do not respond to the best available medical therapy today, even when high doses are used, causing considerable side effects.

Ulcerative colitis typically causes symptoms of bloody diarrhea and faecal urgency. Inflammation involves the rectum, and can extend in a continuous manner to parts of, or the entire proximal colon. Histological examination reveals an infiltrate of chronic inflammatory cells, which are restricted to the superficial layers of the colonic mucosa. Granulomata are not a feature. Crohn's disease is rather more pleomorphic. It is characterized pathologically by discontinuous segments of transmural inflammation, which can affect any part of the gastrointestinal tract from mouth to anus, but most commonly involve the ileocaecal region. Granulomata are a histological hallmark but the clinical features are rather variable depending on the site of bowel involvement. If the colon is inflamed, the symptoms can exactly mimic those of ulcerative colitis.

It is obvious that a possibility to clinically distinguish UC from colonic CD at an early stage would provide enormous benefits for both the patient and the physician. It would permit the design of accurate treatment regimes, prevent the use of unnecessary medications and reduce treatment costs.

Irritable bowel syndrome, or IBS, is also a gastrointestinal disorder in which the smooth muscles of the intestinal wall become hypersensitive to triggers such as stress or even coffee and cause bloating, nausea, vomiting and constipation or diarrhoea. Unfortunately, as many of the symptoms are similar to those of IBD, IBS is often misdiagnosed.

There are some five millions cases of IBS in the USA alone. Enabling to distinguish IBS from the more serious IBD cases would reduce the number of unnecessary visits to the doctor by IBS patients who are suspected to have IBD. It would also help the doctor to choose the most appropriate type of treatment.

The patent literature indicates that the available methods for distinguishing between different forms of IBD, and in particular the differentiation between UC and CD, apart from the above given examples of different examination procedures, have been focused on antibody based methods.

For example WO 03/036262 describes a method and kit for the differentiation of CD from other gastrointestinal illnesses, such as UC and IBS, using the presence of faecal anti-*Saccharomyces cerevisiae* antibodies (ASCA) as a marker for Crohn's disease are provided. The kit includes an enzyme-linked immunoassay or other immunoassay that utilizes antibodies specific to human immunoglobins for the measurement of total endogenous ASCA in a human faecal sample.

WO01/58927 describes diagnostic methods for detecting diseases associated with an autoantigen response to hTM in affected tissue, and in particular UC.

Another intensively studied antibody approach (see U.S. patents 5,691,151; 5,830,675; and 6,033,864) are the autoantibodies to neutrophils (atypical perinuclear anti-neutrophil cytoplasmic antibodies) P-ANCAs. They are found mainly in UC (50-67%) but also in CD (6-15%). The combination of ANCAs and ASCAs was thought to be of good help in patients with indeterminate colitis. But a remarkable finding was that about 50% of the patients with indeterminate colitis are seronegative patients. This means that they do not develop antibodies for ASCAs and ANCAs and thus cannot be diagnosed for, in average, nearly 10 years (Bossuyt X., Clin Chem 2006, 52:2, 171-181)

WO 2004/001073 describes the seven genes referred in the present invention as diagnostic markers for UC. The expression of these markers on RNA level is determined in mucosal biopsies from the colon.

### Summary of the invention

There remains a need for improved methods for the accurate, rapid and reliable screening of patients reporting gastrointestinal problems, and to distinguish between IBD and non-IBD patients, e.g. patients suffering from IBS. It would be particularly desirable, both from a patient and a hospital perspective, if a first screening could be performed before the patient is remitted to ileocolonoscopic examination.

There is also a need for improved diagnosis of ulcerative colitis, in particular in the context of distinguishing between ulcerative colitis and Crohn's disease in IBD patients.

One aim of the present invention is to make available methods as claimed for these purposes. One particular aim is to make available a method which makes it possible to reach a reliable diagnosis at an early stage of the disease. Another aim is to make available a method for a reliable and yet simplified and less invasive method to follow the response to treatment and confirming the improvement in a patient undergoing treatment.

Another aim is to make it possible to perform a first screening using non-invasive or minimally invasive sampling techniques, in contrast to the biopsies or ileocolonoscopic examination normally used. Also the barium enema examination should be classified as an invasive procedure, due to the considerable discomfort and strain it exerts on the patient. It is also an aim to make available methods as outlined in the claims for distinguishing between IBD and IBS, and CD and UC also in difficult cases, where the clinical picture can be very similar.

Further aims underlying the invention, as well as the solutions offered by the invention and the associated advantages will become evident to a skilled person upon study of the description, examples and claims.

The present inventors have surprisingly found that a differentiation between IBD and IBS, and in the case of having determined that the patient suffers from IBD, a further distinction between UC and CD, is made possible by a multi-gene approach where the gene expression profiles are studied in a sample taken from the human body, preferably taken from faeces or blood of a patient, or a biopsy sample obtained from an inflamed area in the intestines.

The present invention is based on the identification of potential marker genes which either collectively or in sub-groups, form the basis for a distinction between IBD and IBS.

One embodiment of the invention, experimentally confirmed, is that the quantification of the expression levels of a number of specific genes and the corresponding proteins can be utilized in accurately and simply determining, using samples from faeces or blood, whether the patient is suffering from IBD, and in a follow up analysis using a biopsy, determine if the patient is afflicted with UC or CD

More specifically, methods are provided that allow the detection of seven (7) distinct markers, singularly or in sub-groups thereof, using gene specific oligonucleotides or antibodies for quantification of the expression levels of said markers. The oligonucleotides or antibodies are designed to recognize specifically the corresponding gene sequence or marker protein. An assay and kit for the detection and monitoring expression status of said seven marker genes or sub-sets thereof in a biological sample are disclosed. The marker genes are (Accession numbers within parenthesis):
SLC6A14 (NM_007231); SLC26A2 (NM_000112); GRO-1 (M_001511); MMP-7 (BC003635); MAP-17 (NM_005764); RegIV (BC017089) also known as GISP, and Vanin-1 (NM_004666).

Results obtained by the inventors indicate that Vanin-1 is a preferred marker gene when analyzing RNA in a blood sample. Also the markers SLC26A2, MAP-17 show utility in blood.

The assay is preferably a method and kit for the quantification of RNA based on PCR-methods.

Alternatively, the assay is a non-culture, antibody-based assay for the detection of said marker proteins.

Another embodiment of the present invention is thus a method for effectively detecting UC or CD in an early stage using antibodies specifically binding to proteins expressed by the human genes SLC6A14, SLC26A2, GRO-1, MMP-7, MAP-17, Reg IV and Vanin-1 in a sample taken from the human body. Results obtained by the inventors indicate that GRO-1 and RegIV are the preferred marker proteins when the source of the sample is human faeces, and Vanin-1, GRO-1 and MMP-7 when the sample is blood.

Another embodiment of the present invention is a method for diagnosing UC and CD by measuring the level of expression of SLC6A14, SLC26A2, GRO-1, MMP-7, MAP-17, Reg IV and Vanin-1 proteins in a sample by an antigen-antibody binding reaction using an antibody specifically binding to a protein expressed from human genes encoding SLC6A14, SLC26A2, GRO-1, MMP-7, MAP-17, Reg IV and Vanin-1.

Further embodiments of the invention are methods for the determination of a patient's response to a treatment, the rate of improvement during treatment and for estimating the prognosis for recovery.

### Brief Description of the Drawings

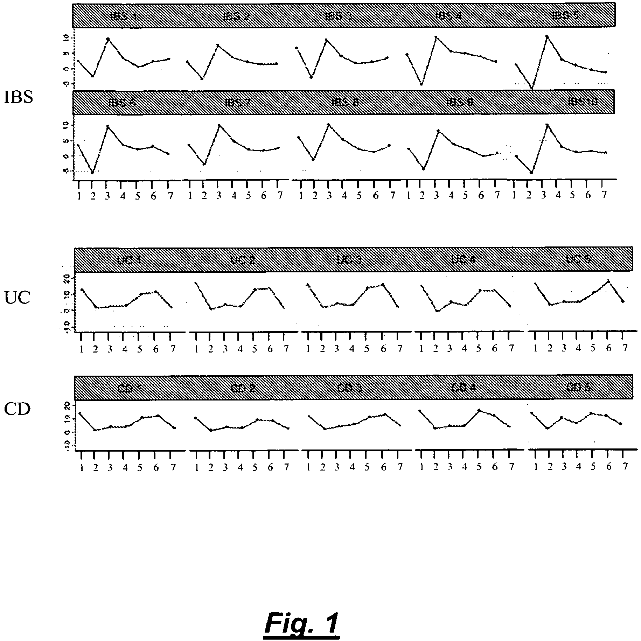

The invention will be disclosed in detail in the following description, examples and drawings, in which
Figure 1 shows expression profiles of 10 IBS and 10 IBD cases derived from real-time analysis of faeces specimen. The seven different markers are plotted on the x-axis, and the corresponding delta Ct values of the PCR analysis are plotted on the y-axis (1=SLC6A14; 2=SLC26A2; 3=GRO1; 4=MMP-7; 5=MAP17; 6=GISP; 7=Vanin-1).

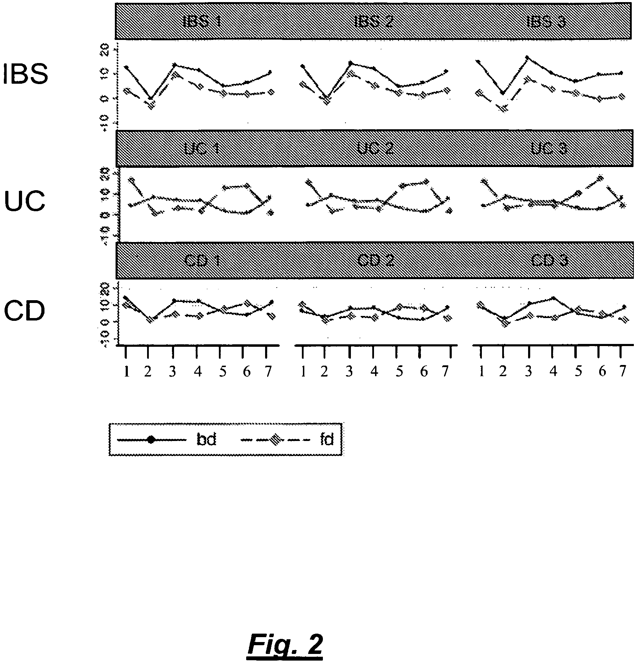

Figure 2 shows the expression profiles in faeces and biopsy samples derived from real-time PCR analysis. The seven different markers are plotted on the x-axis, and the corresponding delta Ct values of the PCR analysis are plotted on the y-axis (1=SLC6A14; 2=SLC26A2; 3=GRO1; 4=MMP-7; 5=MAP17; 6=GISP; 7=Vanin-1). [bd=biopsy derived; fd= faeces derived]

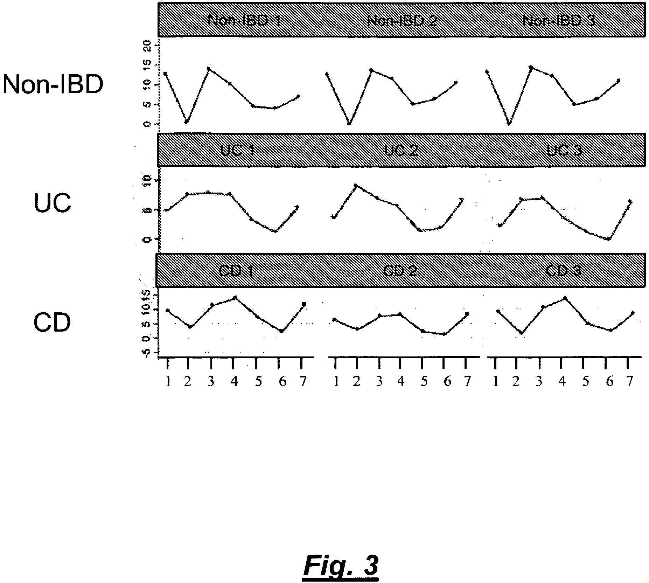

Figure 3 shows the expression profiles in biopsy samples derived from real-time analysis. The seven different markers are plotted on the x-axis, and the corresponding delta Ct values of the PCR analysis are plotted on the y-axis (1=SLC6A14; 2=SLC26A2; 3=GRO1; 4=MMP-7; 5=MAP17; 6=GISP; 7=Vanin-1).

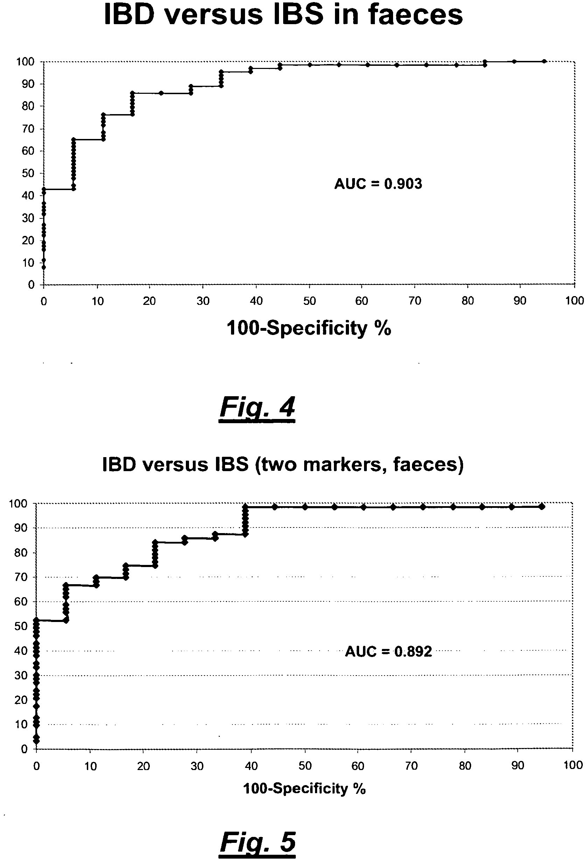

Figure 4 shows a ROC plot as a result of the real-time analysis of 18 non-IBD and 63 IBD patients with all seven markers followed by an analysis with a classification algorithm. The area under the ROC curve (AUC) of 0.903 illustrates the high discriminative potential of all the markers together.
Figure 5 shows a ROC plot as a result of the real-time analysis of 18 non-IBD and 63 IBD patients with only two markers (GRO-1 and Reg IV) followed by an analysis with a classification algorithm. The area under the ROC curve (AUC) of 0.892 illustrates the high discriminative potential of these markers alone.

### Detailed description

Before the present invention is disclosed and described, it is to be understood that one skilled in the art will appreciate readily that the present invention is well adapted to carry out the objects and obtain the ends and advantages mentioned, as well as those objects, ends and advantages inherent herein. The present examples, along with the methods, procedures, treatments, molecules, and specific compounds described herein are presently representative of preferred embodiments, are exemplary, and are not intended as limitations of the scope of the invention.

It is also to be understood that the terminology used herein is for purposes of describing particular embodiments only, and is not intended to be limiting. It must be noted that, as used in the specification and the appended claims, the singular forms "a," "an" and "the" include plural referents unless the context clearly dictates otherwise. Thus, for example, reference to "a sequence" includes more than one such sequence, and the like.

In this specification and in the claims which follow, reference will be made to a number of terms which shall be defined to have the following meanings:
As used herein, the term "complementary DNA primer" means an oligonucleotide, which anneals to the RNA template in a particular orientation to allow for the synthesis of a nascent DNA strand in the presence of reverse transcriptase in the biological sample under the conditions described herein.
Also as used herein, the "condition" under which a DNA strand is synthesized include the presence of nucleotides, cations and appropriate buffering agents in amounts and at temperatures, such that the RNA template and the DNA primer will anneal and oligonucleotides will be incorporated into a synthesized DNA strand if reverse transcriptase is not inhibited by the reverse transcriptase inhibitor drug. Exemplary conditions are set forth in the examples below. The described conditions have been optimized from other known RT/cDNA synthesis protocols. It is generally known that other conditions can be established for optimization of a particular reverse transcriptase reaction on the basis of protocols well known to one of ordinary skill in the art.

As used herein, the term "primer pair" refers to two primers, one having a forward designation and the other having a reverse designation relative to their respective orientations on a double-stranded DNA molecule which consists of a sense and antisense sequence, such that under the amplification conditions described herein, the forward primer anneals to and primes amplification of the sense sequence and the reverse primer anneals to and primes amplification of the antisense sequence. Primers can be selected for use in the amplification reaction on the basis of, having minimal complementarity with other primers in the reaction (to minimize the formation of primer dimers) and having Tm values with the range of reaction temperatures appropriate for the amplification method, preferably PCR. In addition, primers can be selected to anneal with specific regions of the RNA template such that the resulting DNA amplification product ranges in size from 100 to 500 base pairs in length and most preferably around 300 base pairs in length.

As used herein, the terms "detecting" or "detection" of the amplified DNA refers to qualitatively or quantitatively determining the presence of the amplified DNA strand, which is only synthesized if reverse transcriptase is resistant to the reverse transcriptase inhibitor drug added to the assay mixture. The amplification of the synthesized DNA can be detected by any method for the detection of DNA known in the art. For example, detection of the amplified DNA can be by Southern blot hybridization assay, by visualization of DNA amplification products of specific molecular weight on ethidium bromide stained agarose gels, by measurement of the incorporation of radiolabeled nucleotides into the synthesized DNA strand by autoradiography or scintillation measurement.

In the method of the present invention, PCR amplification is accomplished by pre-incubating all PCR reagents and a sample containing a target nucleic acid in the presence of appropriate gene specific primers and a thermostable polymerase enzyme. The resulting reaction mixture is cyclically heated under conditions allowing for the formation and amplification of primer extension products.

The reagents required for PCR are known to persons skilled in the art, and generally include at least two oligonucleotide primers that are sufficiently complementary to conserved regions of the target nucleic acid to hybridise thereto, four different nucleoside triphosphates, a thermostable polymerisation agent and any requisite cofactors for the polymerisation agent. Preferred nucleoside triphosphates are the deoxyribonucleoside triphosphates dATP, dCTP, dGTP and dTTP or dUTP, collectively termed dNTPs. Nucleoside triphosphates are commercially available.

Primers include naturally occurring or synthetically produced oligonucleotides capable of annealing to the target nucleic acid and acting as the point of initiation of nucleic acid synthesis under appropriate conditions, i.e., in the presence of nucleoside triphosphates, a polymerisation agent, suitable temperature, pH and buffer. The primers have sequences sufficiently complementary to the target nucleic acid to hybridise thereto, and are of sufficient length, typically from 10-60 nucleotides, to prime the synthesis of extension products in the presence of a polymerisation agent. Primers can also be produced synthetically by automated synthesis by methods well known to one of ordinary skill in the art.

Design considerations for primers are well known in the art. Primers are selected to be substantially complementary to the sequences of the strands of the specific nucleic acid to be amplified, such that the extension product synthesized from one primer, when separated from its complement, can serve as a template for the extension product of the other primer. Preferably, the primers are exactly complementary with the target region. It is underlined that the primer pairs given in the present specification, examples and claims can be replaced by functionally equivalent primers, exhibiting specificity to the marker genes, without departing from the scope of the invention.

The present inventors collected faeces samples (stool samples) of 33 UC and 24 CD for analysis with real-time PCR to determine if they could discriminate between UC and CD as described for biopsy material. Surprisingly, the inventors found that the expression profiles of these markers were substantially the same for UC and CD in the faeces specimen. As control samples, some IBS cases were used. These exhibited a different expression pattern for the genes as the two major forms of IBD. The inventors analyzed in total 18 IBS cases against the 63 IBD cases. The results in Fig. 1 show a clear difference in the expression profile between IBS and IBD for 10 IBS and 10 IBD cases.

Another surprising fact was that genes which were up-regulated for UC in biopsies were now down-regulated in faeces samples (marker 6 and also to some extent marker 5). Also marker 2 (SLC26A2) was originally down-regulated in UC but now found to be up-regulated compared to marker 1 (SLC14A6). In Figure 2, the expression profiles of faeces and biopsy samples of 3 IBS, 3 UC and 3 CD patients are shown.

In the case of the IBS patients the expression profile of the genes does not change between the different specimens. However, when the profiles in the UC cases are compared, an opposite trend of the two curves can be observed, especially in terms of the contrary expression of markers 2, 5 and 6. Also for CD, the expression pattern is changed in the biopsies (see fig 3).

Solute carrier (SLC) proteins comprise of a very large family of energy dependent transport molecules and have critical physiological roles in nutrient transport and may be utilized as a mechanism to increase drug absorption. However, there is limited understanding of these proteins at the molecular level due to the absence of high-resolution crystal structures.

In total, 1-2% of adults and 6-8% of children suffering from kidney stones have cystinuria, a defect in the transport of amino acids, which leads to high concentrations of cystine in the urine. Two genes have been implicated, solute carrier family 3, cystine, basic and neutral amino acid transporter, member 1 (SLC3A1) coding for the protein related to the system of amino-acid transporter, and solute carrier family 7, member 9 (SLC7A9). Both of these solute carriers are believed to be involved in stone formation which may ultimately lead to urinary tract infection and, eventually, renal failure.

The inventors have now identified two known solute carriers (SLC6A14 and SLC26A2) whose expression is significantly altered in IBD.

CXC chemokine growth-related oncogene-alpha (Gro-alpha also known as GRO 1) is as described a cytokine and as such can alter the migratory responses of numerous cell types in local areas of inflammation. It has been described to be over expressed in human inflamed corneas (Spandau *et al.*, 2003) and in addition, it has also been shown that rats chemically induced to exhibit inflammation of the gut show up-regulated levels of GRO 1 (Hirata *et al.*, 2001). Using a cDNA microarray approach, Heller *et al.*, 1997 describes novel participation of the chemokine Gro alpha in rheumatoid arthritis and inflammatory bowel disease. Keiichi *et al.*, 2006 reported increased circulating concentrations of Growth-Related Oncogene (GRO)-α in patients with IBD. While it is described in Isaacs *et al.*, 1992, that expression of GRO1 in UC is higher than that seen in CD, here it has been demonstrated that there exists an inverse correlation of UC verses CD with respect to GRO 1 expression levels. Lastly Lawrence *et al.*, 2001 describes identifying GRO 1 as being up-regulated in UC, but the design of the study was such that biopsy samples where pooled before analysis, therefore is was not possible to know whether GRO 1 was up-regulated in more than 1 patient.

Matrilysin or (matrix metalloproteinase-7) was first discovered in the involuting rat uterus; it has also been known as uterine metalloproteinase, putative metalloproteinase (Pump-1), and matrix metalloproteinase 7 (MMP-7). It is the smallest member (28 kDa) of a family of 15 MMPs that together are able to degrade most of the macromolecules of the extra cellular matrix. This family is briefly reviewed; all members are zinc metalloproteinases that occur in zymogene form with the active site zinc blocked by cystine. Matrilysin can degrade a wide range of gelatins, proteoglycans, and glycoproteins of the matrix and can activate several other MMPs including collagenase (reviewed in Woessner, 1996).

Matrilysin is frequently expressed in various types of cancer including colon, stomach, prostate, and brain cancers. Previous studies have suggested that matrilysin plays important roles in the progression and metastasis of colon cancer. Recently it has been described by Newell *et al.*, 2002 that there is an increase of matrilysin expression at different stages of UC-associated neoplasia. This work however does not determine whether such increased expression is a result of UC or rather due to the presence of neoplasia.

Membrane associated protein 17 (MAP-17) or otherwise known as DD96 was originally identified as up-regulated in carcinomas with a potential role in modulating cell replication and tumor growth. Newer findings showed that it is also plays a role in surface expression of scavenger receptors class B type I in liver by regulating PDZK1 resulting in a new name SPAP for small PDZK1-associated protein (Identification of small PDZK1-associated protein, DD96/MAP-17, as regulator of PDZK1 and plasma high density lipoprotein levels; Silver et al. J Biol Chem 2003, 278 (31), p28528-32).

Reg IV is a secreted protein and member of the Reg multigene family which have been functionally implicated in regeneration, proliferation and differentiation of the pancreas, the liver and the gastrointestinal mucosa. Reg IV in particular is up-regulated in malignancies of the gastrointestinal tract and is associated with intestinal and neuroendocrine differentiation of stomach and gastric carcinomas (colorectal carcinoma, colon adenocarcinomas); (RegIV activates the epidermal growth factor receptor/Akt/Ap-1 signaling pathway in colon adenocarcinomas; Bishnupuri et al., Gastroenterology 2006 130 (1), p137-49).

Pantetheinase (EC 3.5.1.) is an ubiquitous enzyme which *in vitro* has been shown to recycle pantothenic acid (vitamin B5) and to produce cysteamine, a potent anti-oxidant. The enzyme is encoded by the Vanin-1 gene and is widely expressed in mouse tissues. Vanin-1 is a GPI-anchored pantetheinase, and consequently an ectoenzyme. It has been suggested that Vanin/pantetheinase might be involved in the regulation of some immune functions maybe in the context of the response to oxidative stress (Pitari *et al.*, 2000).

Methods to analyse the quality and quantity of the transcribed mRNA are described in the several laboratory handbooks (for example, in Sambrook and Russell, 2001) and are well known for a person skilled in the art. These methods comprise ribonuclease protection, primer extension, northern blotting, dot blot hybridization, and conventional or real time PCR.

Traditionally, the amount of a particular mRNA produced, and thus the activation status of a gene has been measured by northern blotting. Total RNA isolated from a sample is separated by agarose gel electrophoresis, and probed with a complementary DNA probe specific for the gene of interest. In conventional polymerase chain reaction (PCR), the total RNA isolated from the cell or tissue to be analyzed is reverse transcribed into first strand cDNA (RT-PCR), which is then used as a template to amplify a double stranded amplicon with target specific oligonucleotide primers. In both techniques detection is based on detectable labels, such as fluorescent dyes or radioactive isotopes. Also the recently developed techniques known as DNA chips or microarrays are based on hybridization the target DNA to complementary target specific primers, washing out the unbound DNA and quantifying the bound target DNA. Probes and primers used in the hybridization reactions may be designed based on the nucleotide sequence of the marker gene or amino acid sequence of the translated protein, corresponding the marker gene. A convenient quantitative hybridization method for determining variations in the amounts of expressed RNA is described in the International patent application WO 2002/055734.

Preferably, the "marker gene expression" may be quantified with real time PCR, also called quantitative real time PCR. The method follows the general pattern of polymerase chain reaction, but the amplified region of the target DNA is quantified after each round of amplification by using fluorescent dyes, such as SYBR Green that intercalate with double-stranded DNA or modified DNA oligonucleotide probes that fluoresce when hybridized with a complementary DNA. Frequently, real time PCR is combined with reverse transcription to quantify low abundance mRNA. The data can be analysed by computer software, such as Applied Biosystems 7500 or 7500 Fast Real Time PCR Systems, to calculate relative gene expression between several samples, or mRNA copy number based on a standard curve. Relative quantification (RQ) is commonly used to compare expression levels of wild-type with mutated alleles or the expression levels of a gene in different tissues. RQ determines the change in expression of a target gene in a test sample relative to the same sequence in a basal or calibrator sample (a sample used as the basis for comparative results). The calibrator sample can be an untreated control or a sample at time zero in a time-course study. By using an endogeneous or intrinsic control, it is possible to normalize quantification of a cDNA target for differences in the amount of cDNA added to each reaction. Typically, housekeeping genes such as β-actin, glyseraldehyde-3-phosphate dehydrogenase (GAPDH) and ribosomal RNA (rRNA) are used as endogeneous controls, because their expression levels are relative stable. Replicate reactions per sample and an endogeneous control are needed to ensure statistical significance.

The protein specific antibodies are preferably purified from antiserum obtained by immunizing antigen protein in an animal. More preferably, the protein specific antibodies are polyclonal antibodies purified from the serum obtained by immunizing antigen protein in a rabbit.

To synthesize an antibody specifically recognizing a protein, the protein is first acquired. The protein can be synthesized using known amino acid sequences or produced in recombinant protein types by genetic engineering methods. For example, recombinant protein can be prepared by a method comprising preparing an expression vector of expressing the protein in the form of a recombinant protein using the base sequence of the gene set forth in the NIH program GenBank database; obtaining a transformant by transforming the expression vector into *E. coli* to produce recombinant protein; and cultivating the transformant to isolate/purify the human recombinant protein.

Diagnosing IBD (e.g. UC and CD) by an antigen-antibody binding reaction using the specific antibodies for the proteins enclosed in this invention can be made by determining the expression of these proteins in a sample. The level of expression can be detected by technique known in the art, including enzyme-linked immunosorbent assay (ELISA), radioimmunoassay(RIA), sandwich assay, and Western blotting or immunoblotting analysis on a polyacrylamide gel as well as immunohistochemical assays.

As the sample (specimen), tissues, bodily fluids, most preferably blood and faeces, are preferably used.

One embodiment of the invention is a method for use in the differentiation between inflammatory bowel disease (IBD) and irritable bowel syndrome (IBS) in a patient, wherein the expression levels of at least two markers are determined in a sample taken from said patient, said markers chosen from the group comprising the following genes and their corresponding proteins (Table 1):

**Table 1.**

| Gene: | SEQ.ID.NO. | PROTEIN ACC. NO. |
|---|---|---|
| SLC6A14 | NM_007231 | NP_009162 |
| SLC26A2 | NM_000112 | NP_000103 |
| GRO-1 | NM_001511 | NP_001502 |
| MMP-7 | BC003635 | NP_002414 |
| MAP-17 | NM_005764 | NP_005755 |
| RegIV | BC017089 | NP_114433 |
| Vanin-1 | NM_004666 | NP_004657 |

Another embodiment of the invention is a method wherein, in case inflammatory bowel disease is indicated, a differentiation between ulcerative colitis and Crohn's disease is performed by determining the expression levels of at least two markers in a sample taken from said patient, said markers chosen from the group presented in Table 1 above.

Further, according to an embodiment of the invention the disease activity is monitored, and a distinction is made between an active and passive phase of ulcerative colitis or Crohn's disease, based on the expression pattern of said at least one marker; a gene or corresponding protein of fragments thereof. Preferably SLC6A14 and GRO-1 are used, or alternatively or in combination therewith Reg IV and Vanin-1.

In the above methods, said sample is preferably one of a stool sample, blood, plasma, serum and biopsy samples, most preferably a stool sample or a blood sample.

Preferably the determination is first performed in a stool sample, in order to discriminate between IBS and IBD. If IBD is indicated, a further analysis is made on a biopsy sample, in order to discriminate between UC and CD. The analysis of both stool and biopsy samples can be repeated in order to monitor the progression of the disease or the recovery of the patient.

According to an embodiment of the invention, the expression levels are determined using at least two antibodies capable of identifying said at least two proteins or a fragment thereof, contacting at least a portion of said sample with said at least two antibodies; and monitoring the extent of reaction between the contacted sample and the antibodies.

According to an embodiment of the invention, the contacting and monitoring steps are carried out by extracting the proteins from the sample and conducting an assay to determine the quantity of at least two of the listed proteins therein.

Preferably the antigen-antibody binding reaction is detected by any one technique selected from the group consisting of enzyme-linked immunosorbent assay (ELISA), radioimmunoassay (RIA), sandwich assay, Western blotting, immunoblotting analysis or a immunohistochemistry method.

Alternatively, the contacting and monitoring steps are carried out by immunohistochemically reacting the patient sample and the antibodies and then detecting the reactions in the sample.

According to the invention, the antibodies are polyclonal or monoclonal.

Also disclosed is a kit suitable for use in differentiating between inflammatory bowel disease and irritable bowel syndrome in a patient, said kit comprising at least two antibodies capable of identifying at least two proteins or protein fragments chosen from the group comprising the proteins presented in Table 1 above, and means for detecting the extent of the reaction of the antibodies with a sample.

Further, a kit suitable for differentiating between ulcerative colitis and Crohn's disease is disclosed preferably said kit comprises at least two antibodies capable of identifying at least two proteins or protein fragments chosen from the group comprising the proteins presented in Table 1 above, detecting the extent of the reaction of the antibodies with the proteins directly in bodily fluids wherein the antibodies are coupled to a solid matrix, and further containing instruction for use.

In a kit as disclosed herein, the expression pattern of said at least two proteins is compared to a standard pattern in order to determine whether a patient suffering from ulcerative colitis or Crohn's disease suffers from said disease in its active or passive form.

Further, the kit includes the protein, protein fragment, or peptide provided as a control in a known quantity.

The kit is suitable for use in a competitive immunoassay, and further comprises a solid phase, and the protein, protein fragment or peptide fragment is bound to the solid phase. Said solid phase preferably comprises one or more of nitrocellulose, cellulose, glass, plastic, microtitre plates and wells, such as microtitre wells.

Preferably, the ELISA technique using SLC6A14, SLC26A2, GRO-1, MMP-7, MAP-17, Reg IV and Vanin-1 protein specific antibodies is carried out through the following steps:
1) placing a specific antibody into a reactor coated with a sample and a control group to induce an antigen-antibody reaction;
2) detecting an antigen-antibody reaction product using a secondary antibody-label conjugate and a color-developing substrate solution of a labeling substance; and
3) comparing the detection result of the sample with that of the control group.

A large amount of the sample can be analyzed using known technique such as ELISA, biological microchip or automated microarray system. The biological micro chip can detect an antigen for the specific antibody protein by fixing the specific antibody protein on a biological microchip, causing a reaction between the same and a sample collected from an individual.

Also, a kit for diagnosing UC and CD comprising the combination of at least two of these genes using antibodies specifically reacting with the chosen at least two genes is disclosed.

The diagnostic kit comprises a solid phase or carrier, such as a wicking membrane. Example of materials suitable for such a wicking membrane include, but are not limited to nylon, polyester, cellulose, polysulfone, polyvinylidene difluoride, cellulose acetate, polyurethane, glass fiber, nitrocellulose, or the like. The solid phase can also be in the form of a microcarrier, particles, membranes, strips, paper, film, pearls or plates, such as microtiter plates.

The diagnostic kit can diagnose UC and CD by quantitatively and qualitatively analyzing an antigen of the antibody protein by the antigen- antibody binding reaction. The antigen-antibody binding reaction can be detected by technique generally known in the art, including ELISA, RIA, sandwich assay, Western blotting on a polyacrylamide gel, and immunoblotting analysis. For example, the diagnostic kit can be provided to be used for ELISA using a 96-well microtiter plate coated with recombinant monoclonal antibody protein. Another example would be the use of diagnostic dipsticks for faeces or blood samples.

The controls contained in the diagnostic kit include positive controls and negative controls.

The antibodies used in the diagnosis of the different samples can be polyclonal or monoclonal, and can be made by methods now well-known in the art. For example, polyclonal antibodies made to the extract of Example 2 or 3 can be produced by immunizing an animal such as a rabbit and then purifying the antibodies according to conventional practices, while monoclonal antibodies to said extracts can be made, for example, according to the method of Kohler and Milstein (Nature (1975) 256:495) by immunizing an animal such as a mouse, extracting splenocytes from the spleen thereof, fusing them with mouse myeloma cells to make hybridomas, and screening and subcloning the hybridomas, according to well know practices.

In a preferred embodiment, the immunoassay used in the method of the present invention is of the ELISA sandwich type. In a more preferred embodiment, the ELISA sandwich immunoassay involves the pretreatment of the sample with a specific buffer (clearing buffer) containing a pancreatic cholesterol esterase for decreasing interference of sample circulating lipids in protein quantification. In another preferred embodiment the immunoassay is a Western blot assay (specially preferred with an internal control).

As the secondary antibody labeling substance, known labeling substances inducing color development are preferably used, and examples the labeling substance useful in the present invention include horseradish peroxidase (HRP), alkaline phosphatase, colloidal gold, fluorescein such as poly L-lysine-fluorescein isothiocyanate(FITC) or rhodamine-B-isothiocyanate (RITC), and a dye. In the present invention, goat anti-rabbit IgG-HRP conjugate (IgG-HRP conjugate), for example, is used.

Preferably, the chromogen used varies according to the labeling substance involving the color development, and usable examples thereof include 3,3', 5, 5'-tetramethyl bezidine (TMB), 2,2'-azino-bis (3-ethylbenzothiazoline-6-sulfonic acid) (ABTS), and o-phenylenediamine (OPD).

More preferably, the chromogen is provided in a state in which it is dissolved in a buffer solution (0.1 M NaAc, pH 5.5). The chromogen such as TMB is decomposed by HRP used as the labeling substance of the secondary antibody conjugate to produce a color-developing precipitate. The level of precipitation of the color-developing precipitate is observed by the naked eye, thereby determining the presence of a protein antigen.

The washing solution preferably include a phosphate buffer solution, NaCl, and Tween 20, more preferably a buffer solution containing 0.02 M phosphate buffer solution, 0.13 M NaCl, and 0.05% Tween 20. Following antigen-antibody binding reaction, an appropriate amount of the washing solution is supplied into the reactor having undergone the reaction between the secondary antibody and the antigen-antibody conjugate. Washing with the washing solution is repeatedly performed 3 to 6 times. 0.1 % BSA containing phosphate buffer is preferably used as the blocking solution and a 2 N sulfuric acid solution is preferably used as the enzymatic reaction stopping solution.

The method of diagnosing UC and CD in an early stage by detecting SLC6A14, SLC26A2, GRO-1, MMP-7, MAP-17, Reg IV and Vanin-1antigens in a specimen sample using the diagnostic kit will now be described. Antibodies and specimen samples in the positive and negative control groups are reacted, respectively, and washed with the washing solution. Then, the secondary antibody conjugate labeled with a labeling substance producing a color by the reaction with the substrate is added to the resultant product and washed again with the washing solution. Then, the substrate containing solution is added to the resultant production to induce color development. Then, the absorbance at 450 nm is measured. Here, the average absorbance of the standard antigen solution A should be greater than or equal to 0.000 and less than or equal to 0.200. The average absorbance of the standard antigen solution F should be greater than or equal to 1.200 and less than or equal to 3.000. The mean value between the absorbance values of the standard antigen solutions A and F is set as a cut-off value to then be used to determine samples as positive or negative samples. When the absorbance of a sample is greater than that of the standard antigen solution F, the sample is diluted and the absorbance thereof is then measured again. The sample having absorbance above the cut-off value is identified as being positive, and the sample having absorbance below the cut-off value is identified as being negative.

It was confirmed that the UC/CD diagnostic kit containing the human specific antibodies for SLC6A14, SLC26A2, GRO-1, MMP-7, MAP-17, Reg IV and Vanin-1, which is a new immunological diagnostic tool using patient's blood or faeces (biopsy), had higher accuracy and reproducibility. Conventionally, an combination between endoscopy, histology, disease history and P-ANCA antibodies is used for diagnosis of IBD which is, however, not very high in accuracy (Bossuyt, X., Clin Chem 52 2006). However, the test for early diagnosis of UC and CD according to the present invention showed a diagnosing accuracy of about 95 to 97%, which is statistically significantly higher than the conventional tests. Therefore, the diagnosing method according to the present invention can be very advantageously used for early diagnosis of UC and CD and diagnosis of IBS because they have high accuracy and reproducibility.

### Examples

The present invention will now be illustrated in detail by the following examples, without in anyway being limited in scope to the specific embodiments described herein.

### Example 1. Tissue collection and storage

Colon tissue biopsies, faeces and blood samples were taken from patients suffering from UC, CD or IBS and from healthy volunteers.

### 1.1 For RNA work:

Faeces samples were taken from patients suffering from UC, CD or IBS (5 to 10 patients each). The faeces samples were mixed in a 1:1 ratio with RNAlater® solution (Ambion Inc./Applied Biosystems) and kept at RT until use.

### 1.2 For protein work:

Faeces samples were taken from patients suffering from UC, CD or IBS. The faces samples were directly frozen at -20°C until use. Alternatively, the samples are mixed with appropriate buffer in a 1:1 ratio.

### Example 2. RNA extraction from faeces samples for Real-time PCR analysis

### 2.1 RNA isolation:

Total RNA from all patient faeces samples was isolated by first homogenizing the biopsies using a Pellet Pestle Motor Homogenizer according to the manufacturer's protocol (Kimble / Kontes, Kimble Glass Inc.). From the homogenate, total RNA was isolated using Qiagen RNeasy® Kit as according to manufactures guidelines (Qiagen Nordic AB).

### 2.2 cDNA synthesis:

Two micrograms of each RNA sample was used for a first strand cDNA synthesis using 10pM of the Oligo-dT-primer VN-T20 (5'- TTT TTT TTT TTT TTT TTT TTN V-3'). The buffer, deoxynucleotide triphosphates (dATP, dCTP, dGTP and dTTP) and the enzyme reverse transcriptase (Superscript II) were supplied by Invitrogen and the reactions were performed according to the manufactures guidelines. The reaction mixture for first strand synthesis excluding the enzyme was preincubated for 10 min at 65°C in a PCR machine (PCR sprint from Hybaid), chilled on ice, and then the enzyme Superscript II was added and incubated for 1,5 hour at 42°C in a PCR machine.

### 2.3 Realtime PCR analysis of cDNA derived from faeces samples:

Real time PCR analysis was performed on an applied biosystems 7500 PCR machine using 1:10 diluted 1st strand cDNA material derived from each faeces sample and the SYBR Green Mastermix from Applied Biosystems according to the manufacturer's recommendations. Primers were designed to amplify the seven marker genes and internal control, resulting in fragments ranging 106-160bp in size. (Small fragments are necessary as fragments over 400bp tend not to give reliable signals). Using software provided for use with the real-time PCR analysis (SDS 4.0; relative quantification assay), so called "delta Ct" values were obtained for all makers for each faeces sample analyzed.

For this type of analysis special real time PCR primers were used giving the indicated fragment size:

**Table 2.**

| | | | | | |
|---|---|---|---|---|---|
| 1. | γ-actin | 150bp | 5. | MMP7 | 140bp |
| 2. | SLC6A14 | 145bp | 6. | MAP 17 | 143bp |
| 3. | SLC26A2 | 106bp | 7. | GISP | 160bp |
| 4. | GRO1 | 125bp | 8. | Vanin-1 | 144bp |

The expression profile was then determined by plotting the delta Ct values for each marker against each marker and analyzing the graph (see results).

The primers, forward and reverse, used in the experiments are presented in Table 3.

**Table 3.**

| **Gene** | **forward primer** | **reverse primer** |
|---|---|---|
| SLC6A14 | 5'- GAG CAA AGA GGT GGA TAT TCT GGC - 3' | 5'- CTC CCC AGT CAG GGT ATG GAA TTG - 3' |
| SLC26A2 | 5'- CAC CTA AAG CTA TTA TGC AGG AGG - 3' | 5'- CTC CTC AAT TCA TGA CCT GTG GGC - 3' |
| GRO-1 | 5'- GCC AAT GAG ATC ATT GTG AAG GCA - 3' | 5'- CAA CAT GAG AAA TGT TGA CCA CAC - 3' |
| MMP-7 | 5'- CAC TGT TCT TCC ACT CCA TTT AGC - 3' | 5'- GAC ATC TAC CCA CTG CAA GTA TAG - 3' |
| MAP17 | 5'- GTT CCT GGT CCT CGT TGC AAT CGC - 3' | 5'- CCA TCG AAG AGT ACC TTC CAT CTG - 3' |
| Reg IV | 5'- GGT GAT ATC ATC ATG AGA CCC AGC - 3' | 5'- CTT TAA ACT CAG GAT AGA TGC CAG - 3' |
| Vanin-1 | 5'- CCA ACT GAC TGA TAG ACT CTG AGC - 3' | 5'- GGC ATA GAT CAC TAC TGC AAG TGC - 3' |
| γ-Actin | 5'- GTG CAG GGT ATT AAC GTG TCA GGG - 3' | 5'- CCA ACT CAA AGC AAG TAA CAG CCC ACG G - 3' |

The RNA data show that GRO-1 and Reg IV are a good combination for discrimination of IBD and IBS on RNA level in faeces (See e.g. Fig. 5). Further, RNA data from blood samples shows that Vanin-1 has a good discriminating potential has. The markers SLC26A2, MAP-17 and Vanin-1 are well detectable in blood on the RNA level. The markers GRO-1, MMP-7 and RegIV. SLC14A6 were detectable, but on a lower level, when expressed in blood on RNA level.

From the fact that MMP-7, RegIV and GRO-1 are secretory proteins, the inventors conclude that the expression on protein level would be easier to detect, and this could be used to confirm the performance of the blood RNA data.

Regarding RNA in blood, it should be noted that the discrimination of IBD and IBS was reliable already using only one marker, Vanin-1. The AUC was 0.867.

### Example 3. Protein extraction

### 3.1 Protein extraction from biopsy samples

The obtained fixed biopsy samples have to be centrifuged, the fixative removed and washed with PBS. Then extraction buffer (100mM potassium phosphate pH 7,8; 1 mM DTT; 0,5mM PMSF) was added and the sample homogenized using a Pellet Pestel Homogenizer according to the manufacturer's protocol. Then 0,1-0,2% of Triton-X-100 was added and the mixture incubated for 5 min at 4°C to lyse the cells. After centrifugation of 5min at 4°C the supernatant (=extract) was transferred into a new tube and the extract stored after shock freezing at -80°C until usage.

### 3.2 Protein extraction from faeces samples

Extraction buffer (100mM potassium phosphate pH 7,8; 1 mM DTT; 0,5mM PMSF) and glass beads were added to the faeces samples and thoroughly vortexed. Then 0,1-0,2% of Triton-X-100 was added and incubated for 5 min at 4°C to lyse the cells. After centrifugation during 5 min at 4°C, the supernatant (=extract) was transferred into a new tube and the extract stored at -80°C until use

### 3.3 Protein extraction from blood

To the frozen serum the same amount of 2x extraction buffer (200mM potassium phosphate pH 7,8; 2mM DTT; 1 mM PMSF) plus 0,2-0,4% of Triton-X-100 was added and the mixture incubated for 5 min at 4°C to lyse the cells. After centrifugation during 5min at 4°C, the supernatant (=extract) was transferred into a new tube and the extract shock frozen and stored at -80°C until usage.

Instead of extracting the proteins from the blood or the serum, the serum can be used directly to coat the ELISA plates.

### Example 4. Western Blot Analysis of the specimen samples with polyclonal antibodies

For each patient and specimen sample, the protein extract (standardized to 10 µg of total protein/lane) were separated eight times by 12 or 15% denaturing SDS-PAGE and transferred to a polyvinylidene difluoride membrane (Hybond-P; 0.8 mA/cm.sup.2 for 60 min; Amersham Pharmacia Biotech) by semidry blotting using an electroblotter (Bio-Rad). The membrane was then dissected into eight stripes for reaction with specific antibodies for the proteins of PROT. ID. 1 - 7 and beta-tubulin. Antibodies were diluted in blocking buffer. Membranes were subsequently washed, incubated with ECL-Plus Detection Reagent, and exposed to Hyperfilm ECL (both from Amersham Pharmacia Biotech). Primary antibodies directed against Reg IV and MMP-7 were obtained from Abcam, GRO-1 from RDS and SLC6A14, SLC26A2, MAP-17 and Vanin-1 were synthesized by commercial suppliers. Protein contents were normalized by parallel hybridization with an antibody against alpha-tubulin. All Western blots were exposed to film for varying lengths of time, and only films generating subsaturating levels of intensity were selected for densitometrical and statistical evaluation. Linearity was assured in independent experiments by using different amounts of material and multiple film exposures (data not shown).

As a positive control the recombinant proteins of PROT. ID. 1-7 (obtained from the same source as the antibodies) were also analysed in the same way.

### Example 5: ELISA analysis using polyclonal antibodies raised against the marker proteins

First, wells were coated with the sample. Second, ELISA plate wells were coated with the specific polyclonal antibodies to the seven proteins, PROT ID NO 1 - 7. The third step was the detection of the presence of specific antigen-antibody binding in the samples.

### 5.1 Coating with sample on the wells

Protein extracts obtained from colon biopsies, faeces and blood samples were used as specimen samples. To obtain a standard curve for measurement of cut-off values, standard antigen solutions 1 to 7 were prepared with dilutions A, B, C, D, E, F and G at various concentrations of 0ng/ml, 20 ng/ml, 40 ng/ml, 80 ng/ml, 160 ng/ml, 320 ng/ml and 640 ng/ml, respectively.

100 µl of the respective specimen protein samples were distributed to respected 96-well ELISA flat-bottomed plate and reacted at 37°C for 4 hours, followed by washing 4 times with a washing buffer solution (PBS including 0.05% Tween 20, Ph 7.4). Here, the standard antigen solutions prepared above were used as positive control groups and normal rabbits sera were used as negative control groups.

### 5.2 Addition of the polyclonal antibodies

Specific polyclonal antibodies to the proteins of PROT. ID 1-7 were placed in each well coated with the specimen samples, covered with a lid and allowed to stand at 4 C for 16 to 18 hours. The polyclonal antibodies were diluted in 0.5 M carbonate buffer (pH 9.6) in a concentration of 5 µg/ml and 100 µl of the diluted solution was added to each well. As a control group, the normal rabbit serum that was not infected with the proteins was 500-fold diluted in a carbonate buffer solution and distributed to each well (100 µl/well).

Then, the wells of the plate were washed 4 times with a washing buffer solution. In order to block non-specific protein binding sites, a blocking solution (PBS buffer solution (pH 7.4) containing a 2% BSA) was distributed to each well (300pl/well) and allowed to stand at 37°C at 2 hours.

### 5.3 Detection of Antigen-Antibody Complex

100 µl of a 10,000-fold dilution of horseradish peroxidase conjugated goat anti-rabbit IgG secondary antibody was added to each well, and the plate was allowed to stand at 37°C for 1 hour, followed by washing 4 times with a washing buffer solution. Subsequently, 1 mg of 3.3', 5.5'-tetramethylbenzidine (TMB) (Sigma Co., USA) as a substrate was dissolved in 10 ml of a citrate buffer solution (pH 5.0) and 2µl of 35% hydrogen peroxide was added thereto, thereby preparing a substrate solution.100 ul of the prepared substrate solution was distributed to each well and reacted at room temperature for 15 minutes without exposure to light. Thereafter, 50 µl of a 2 N H2SO4 solution was added to stop the reaction and the absorbance at 450 nm was measured.

For each specimen sample tested with one of the proteins of PROT. ID 1-7, the absorbance by the antigen was inferred as the remainder obtained by subtracting the absorbance of wells coated with only the protein as the positive control group and PBS as the negative control group from the absorbance of the sample.

In the same manner, after the absorbance values of the standard solutions were calculated the mean value between the absorbance values of the standard antigen solutions A and F for each protein was set as a cut-off value. The sample having absorbance above the cut-off value was identified as being positive, and the sample having absorbance below the cut-off value was identified as being negative.

Based on the cut-off value, the absorbance values of the respective samples were compared to determine whether they are positive or negative.

### References

KS Bishnupuri LQ, N Murmu, C W Houchen, S Anant, B K Dieckgraefe. Reg IV activates the epidermal growth factor receptor/Akt/AP-1 signaling pathway in colon adenocarcinomas. Gastroenterology 2006;130:137-49.
Bossuyt X. Serologic Markers in Inflammatory Bowel Disease. Clin Chem. 2006; 52:171-181
Heller et al., Discovery and analysis of inflammatory disease-related genes using cDNA microarrays, Proc Natl Acad Sci USA. 1997 Mar 18;94(6):2150-5.
Hirata I, Murano M, Nitta M, Sasaki S, Toshina K, Maemura K, Katsu K (2001). Estimation of mucosal inflammatory mediators in rat DSS-induced colitis. Possible role of PGE(2) in protection against mucosal damage. Digestion. 63 Suppl 1:73-80
Isaacs KL, Sartor RB, Haskill S (1992). Cytokine messenger RNA profiles in inflammatory bowel disease mucosa detected by polymerase chain reaction amplification. Gastroenterology. 103:1587-95
Keiichi Mitsuyama OT, Nobuo Tomiyasu, Koseku Takaki, Asuka Suzuki, Junya Masuds Hiroshi Yamasaki, Atsushi Toyonaga, Michio Sata. Increased Circulating Concentrations of Growth-Related Oncogene (GRO)-α in Patients with Inflammatory Bowel Disease. Digestive Diseases and Sciences 2006;51:173-77
Kohler and Milstein (Nature (1975) 256:495)
Lawrance IC, Fiocchi C, Chakravarti S (2001). Ulcerative colitis and Crohn's disease: distinctive gene expression profiles and novel susceptibility candidate genes. Hum Mol Genet. 10:445-56.
Newell KJ et al., Matrilysin (matrix metalloproteinase-7) expression in ulcerative colitis-related tumorigenesis, Mol Carcinog. 2002 Jun;34(2):59-63.
Pitari G et al.., Pantetheinase activity of membrane-bound Vanin-1: lack of free cysteamine in tissues of Vanin-1 deficient mice, FEBS Lett. 2000 Oct 20;483(2-3):149-54
Silver DL, Wang N, Vogel S. Identification of Small PDZK1-associated Protein, DD96/MAP17, as a Regulator of PDZK1 and Plasma High Density Lipoprotein Levels. The Journal of Biological Chemistry 2003;278:28528-532.
Spandau et al., High expression of chemokines Gro-alpha (CXCL-1), IL-8 (CXCL-8), and MCP-1 (CCL-2) in inflamed human corneas in vivo, Ophthalmol. 2003 Jun;121(6):825-31.
Woessner JF Jr, Regulation of matrilysin in the rat uterus, Biochem Cell Biol. 1996;74(6):777-84.

## Claims

1. An in vitro method for determination of gene expression profiles in a sample taken from a human body in a method for differentiation between inflammatory bowel disease (IBD) and irritable bowel syndrome (IBS) in a patient, wherein at least two marker genes are chosen from the following genes and their corresponding proteins:
| Gene: | SEQ.ID.NO. | PROTEIN ACC. NO. |
|---|---|---|
| SLC6A14 | NM_007231 | NP_009162 |
| SLC26A2 | NM_000112 | NP_000103 |
| GRO-1 | NM_001511 | NP_001502 |
| MMP-7 | BC003635 | NP_002414 |
| MAP-17 | NM_005764 | NP_005755 |
| RegIV | BC017089 | NP_114433 |
| Vanin-1 | NM_004666 | NP_004657 |
and wherein the expression levels of said at least two genes and their corresponding proteins are determined in order to obtain an expression profile and wherein the expression profile is compared to the expression profiles of samples from known IBD and IBS patients.

2. The method according to claim 1, wherein the expression level of all seven markers are determined.

3. The method according to claim 1, wherein one of said at least two markers is SLC26A2 and wherein SLC26A2 is expressed at a lower level in IBD compared to in IBS.

4. The method according to claim 1, wherein one of said at least two markers is GRO-1 and wherein GRO-1 is expressed at a higher level in IBD compared to in IBS.

5. The method according to claim 1, wherein said sample is one of a faeces sample, blood, plasma, serum and a biopsy sample.

6. The method according to claim 5, wherein the sample is a faeces sample and one of said at least two markers is MAP-17 and wherein MAP-17 is expressed at a lower level in IBD compared to in IBS.

7. The method according to claim 5, wherein the sample is a faeces sample and one of said at least two markers is RegIV and wherein RegIV is expressed at a lower level in IBD compared to in IBS.

8. The method according to claim 5, wherein said sample is a faeces sample and said at least two markers are SLC6A14 and GRO-1 and wherein SLC6A14 is expressed at a lower level than GRO-1 in IBD and at a higher level than GRO-1 in IBS.

9. The method according to claim 5, wherein said sample is a faeces sample and said at least two markers are GRO-1 and MAP-17 and wherein GRO-1 is expressed at a higher level than MAP-17 in IBD and at a lower level than MAP-17 in IBS.

10. The method according to claim 5, wherein said sample is a faeces sample and said at least two markers are GRO-1 and RegIV and wherein GRO-1 is expressed at a higher level than RegIV in IBD and at a lower level than RegIV in IBS.

11. The method according to claim 5, wherein the sample is a blood sample and one of said at least two markers is Vanin-1.

12. The method according to claim 1, wherein, in a case where IBD is indicated, the expression profile of at least two of the marker genes is determined in a biopsy sample and used for differentiation between ulcerative colitis (UC) and Crohn's disease (CD) and wherein
SLC6A14 is expressed at a higher level in UC compared to CD;
SLC26A2 is expressed at a lower level in UC compared to CD;
GRO-1 is expressed at a higher level in UC compared to CD;
MMP-7 is expressed at a higher level in UC compared to CD;
MAP-17 is expressed at a higher level in UC compared to CD;
RegIV is expressed at a higher level in UC compared to CD; and
Vanin-1 is expressed at a higher level in UC compared to CD

13. The method according to claim 12, wherein the gene expression profile of at least two markers is used for a distinction between an active and passive phase of ulcerative colitis or Crohn's disease.

14. The method according to claim 13, wherein said at least two markers are SLC6A14 and GRO-1.

15. The method according to claim 13, wherein said at least two markers are RegIV and Vanin-1.

## Patentansprüche

1. In-vitro-Verfahren zur Bestimmung von Genexpressionsprofilen in einer Probe, die einem menschlichen Körper entnommen wurde, in einem Verfahren zur Differenzierung zwischen entzündlicher Darmerkrankung (Inflammatory Bowel Desease, IBD) und Reizdarmsyndrom (Irritable Bowel Syndrome, IBS) bei einem Patienten, wobei mindestens zwei Marker-Gene aus den folgenden Genen und deren entsprechenden Proteinen ausgewählt werden:
| Gen: | SEQ.-ID. Nr. | Protein Zugangs-Nr. |
|---|---|---|
| SLC6A14 | NM_007231 | NP_009162 |
| SLC26A2 | NM_000112 | NP_000103 |
| GRO-1 | NM_001511 | NP_001502 |
| MMP-7 | BC003635 | NP_002414 |
| MAP-17 | NM_005764 | NP_005755 |
| RegIV | BC017089 | NP_114433 |
| Vanin-1 | NM_004666 | NP_004657 |
und wobei die Expressionsniveaus der mindestens zwei Gene und ihrer entsprechenden Proteine bestimmt werden, um ein Expressionsprofil zu erhalten, und wobei das Expressionsprofil mit den Expressionsprofilen von Proben von bekannten IBD- und IBS-Patienten verglichen wird.

2. Verfahren nach Anspruch 1, wobei das Expressionsniveau aller sieben Marker bestimmt wird.

3. Verfahren nach Anspruch 1, wobei einer der mindestens zwei Marker SLC26A2 ist und wobei SLC26A2 bei IBD im Vergleich zu IBS in einem niedrigeren Niveau exprimiert wird.

4. Verfahren nach Anspruch 1, wobei einer der mindestens zwei Marker GRO-1 ist und wobei GRO-1 bei IBD im Vergleich zu IBS in einem höheren Niveau exprimiert wird.

5. Verfahren nach Anspruch 1, wobei es sich bei der Probe um eines aus einer Stuhlprobe, Blut, Plasma, Serum und einer Biopsieprobe handelt.

6. Verfahren nach Anspruch 5, wobei es sich bei der Probe um eine Stuhlprobe handelt und einer der mindestens zwei Marker MAP-17 ist und wobei MAP-17 bei IBD im Vergleich zu IBS in einem niedrigeren Niveau exprimiert wird.

7. Verfahren nach Anspruch 5, wobei es sich bei der Probe um eine Stuhlprobe handelt und einer der mindestens zwei Marker RegIV ist und wobei RegIV bei IBD im Vergleich zu IBS in einem niedrigeren Niveau exprimiert wird.

8. Verfahren nach Anspruch 5, wobei es sich bei der Probe um eine Stuhlprobe handelt und die mindestens zwei Marker SLC6A14 und GRO-1 sind und wobei SLC6A14 bei IBD in einem niedrigeren Niveau als GRO-1 und bei IBS in einem höheren Niveau als GRO-1 exprimiert wird.

9. Verfahren nach Anspruch 5, wobei es sich bei der Probe um eine Stuhlprobe handelt und die mindestens zwei Marker GRO-1 und MAP-17 sind und wobei GRO-1 bei IBD in einem höheren Niveau als MAP-17 und bei IBS in einem niedrigeren Niveau als MAP-17 exprimiert wird.

10. Verfahren nach Anspruch 5, wobei es sich bei der Probe um eine Stuhlprobe handelt und die mindestens zwei Marker GRO-1 und RegIV sind und wobei GRO-1 bei IBD in einem höheren Niveau als RegIV und bei IBS in einem niedrigeren Niveau als RegIV exprimiert wird.

11. Verfahren nach Anspruch 5, wobei es sich bei der Probe um eine Blutprobe handelt und einer der mindestens zwei Marker Vanin-1 ist.

12. Verfahren nach Anspruch 1, wobei in einem Fall, bei dem IBD indiziert ist, das Expressionsprofil von mindestens zwei der Marker-Gene in einer Biopsieprobe bestimmt und für die Differenzierung zwischen Colitis ulcerosa (Ulcerative Colitis, UC) und Crohn'scher Krankheit (Crohn's Desease, CD) verwendet wird und wobei
SLC6A14 bei UC im Vergleich zu CD in einem höheren Niveau exprimiert wird;
SLC26A2 bei UC im Vergleich zu CD in einem niedrigeren Niveau exprimiert wird;
GRO-1 bei UC im Vergleich zu CD in einem höheren Niveau exprimiert wird;
MMP-7 bei UC im Vergleich zu CD in einem höheren Niveau exprimiert wird;
MAP-17 bei UC im Vergleich zu CD in einem höheren Niveau exprimiert wird;
RegIV bei UC im Vergleich zu CD in einem höheren Niveau exprimiert wird;
Vanin-1 bei UC im Vergleich zu CD in einem höheren Niveau exprimiert wird.

13. Verfahren nach Anspruch 12, wobei das Genexpressionsprofil von mindestens zwei Markern für eine Unterscheidung zwischen einer aktiven und einer passiven Phase der Colitis ulcerosa oder der Crohn'schen Krankheit verwendet wird.

14. Verfahren nach Anspruch 13, wobei die mindestens zwei Marker SLC6A14 und GRO-1 sind.

15. Verfahren nach Anspruch 13, wobei die mindestens zwei Marker RegIV und Vanin-1 sind.

## Revendications

1. Procédé *in vitro* de détermination de profils d'expression génique dans un échantillon provenant d'un organisme humain dans un procédé de différenciation entre la maladie inflammatoire de l'intestin (MII) et le syndrome de l'intestin irritable (SII) chez un patient, dans lequel on choisit au moins deux gènes marqueurs parmi les gènes suivants et leurs protéines correspondantes :
| Gène : | SEQ ID N° | N° d'accession de la protéine |
|---|---|---|
| SLC6A14 | NM_007231 | NP_009162 |
| SLC26A2 | NM_000112 | NP_000103 |
| GRO-1 | NM_001511 | NP_001502 |
| MMP-7 | BC003635 | NP_002414 |
| MAP-17 | NM_005764 | NP_005755 |
| Reg IV | BC017089 | NP_114433 |
| Vanine-1 | NM_004666 | NP_004657 |
et dans lequel on détermine les niveaux d'expression desdits deux gènes ou plus et de leurs protéines correspondantes afin d'obtenir un profil d'expression et on compare ce profil d'expression aux profils d'expression d'échantillons provenant de patients MII et SII connus.

2. Procédé selon la revendication 1, dans lequel on détermine le niveau d'expression de l'ensemble des sept marqueurs.

3. Procédé selon la revendication 1, dans lequel l'un desdits deux marqueurs ou plus est le SLC26A2 et dans lequel le SLC26A2 est exprimé dans la MII à un niveau plus bas que dans le SII.

4. Procédé selon la revendication 1, dans lequel l'un desdits deux marqueurs ou plus est le GRO-1 et dans lequel le GRO-1 est exprimé dans la MII à un niveau plus élevé que dans le SII.

5. Procédé selon la revendication 1, dans lequel ledit échantillon est choisi parmi un échantillon de selles, de sang, de plasma, de sérum et un échantillon de biopsie.

6. Procédé selon la revendication 5, dans lequel l'échantillon est un échantillon de selles et l'un desdits deux marqueurs ou plus est le MAP-17 et dans lequel le MAP-17 est exprimé dans la MII à un niveau plus bas que dans le SII.

7. Procédé selon la revendication 5, dans lequel l'échantillon est un échantillon de selles et l'un desdits deux marqueurs ou plus est le RegIV et dans lequel le RegIV est exprimé dans la MII à un niveau plus bas que dans le SII.

8. Procédé selon la revendication 5, dans lequel ledit échantillon est un échantillon de selles et lesdits deux marqueurs ou plus sont le SLC6A14 et le GRO-1 et dans lequel le SLC6A14 est exprimé à un niveau inférieur à celui du GRO-1 dans la MII et à un niveau supérieur à celui du GRO-1 dans le SII.

9. Procédé selon la revendication 5, dans lequel ledit échantillon est un échantillon de selles et lesdits deux marqueurs ou plus sont le GRO-1 et le MAP-17 et dans lequel le GRO-1 est exprimé à un niveau supérieur à celui du MAP-17 dans la MII et à un niveau inférieur à celui du MAP-17 dans le SII.

10. Procédé selon la revendication 5, dans lequel ledit échantillon est un échantillon de selles et lesdits deux marqueurs ou plus sont le GRO-1 et le RegIV et dans lequel le GRO-1 est exprimé à un niveau supérieur à celui du RegIV dans la MII et à un niveau inférieur à celui du RegIV dans le SII.

11. Procédé selon la revendication 5, dans lequel le échantillon est un échantillon de sang et l'un desdits deux marqueurs ou plus est la vanine-1.

12. Procédé selon la revendication 1, dans lequel, en cas d'indication de MII, on détermine le profil d'expression d'au moins deux des gènes marqueurs dans un échantillon de biopsie et on l'utilise pour différencier la colite ulcéreuse (CU) de la maladie de Crohn (MC) et dans lequel
le SLC6A14 est exprimé dans la CU à un niveau plus élevé que dans la MC ;
le SLC26A2 est exprimé dans la CU à un niveau plus bas que dans la MC ;
le GRO-1 est exprimé dans la CU à un niveau plus élevé que dans la MC ;
le MMP-7 est exprimé dans la CU à un niveau plus élevé que dans la MC ;
le MAP-17 est exprimé dans la CU à un niveau plus élevé que dans la MC ;
le RegIV est exprimé dans la CU à un niveau plus élevé que dans la MC ; et
la vanine-1 est exprimée dans la CU à un niveau plus élevé que dans la MC

13. Procédé selon la revendication 12, dans lequel on utilise le profil d'expression génique d'au moins deux marqueurs pour faire la distinction entre une phase active et une phase passive de la colite ulcéreuse ou de la maladie de Crohn.

14. Procédé selon la revendication 13, dans lequel lesdits deux marqueurs ou plus sont le SLC6A14 et le GRO-1.

15. Procédé selon la revendication 13, dans lequel lesdits deux marqueurs ou plus sont le RegIV et la vanine-1.
